## Europäisches Patentamt
## European Patent Office
## Office européen des brevets

(19)

(11) Numéro de publication: **0 227 566**
**B1**

(12) **FASCICULE DE BREVET EUROPEEN**

(45) Date de publication du fascicule du brevet: **01.08.90**

(51) Int. Cl.⁵: **A 61 F 13/08**

(21) Numéro de dépôt: **86440081.7**

(22) Date de dépôt: **23.09.86**

(54) **Chaussette de contention pour membre inférieur.**

(30) Priorité: **26.09.85 FR 8514412**

(43) Date de publication de la demande:
**01.07.87 Bulletin 87/27**

(45) Mention de la délivrance du brevet:
**01.08.90 Bulletin 90/31**

(84) Etats contractants désignés:
**AT BE CH DE GB IT LI LU NL SE**

(56) Documents cités:
**CH-A- 465 762**
**FR-A-1 111 157**
**FR-A-1 124 140**
**FR-A-2 347 922**
**US-A-2 646 797**
**US-A-4 166 460**
**US-A-4 502 158**

(73) Titulaire: **Etienne, Abel**
**29, rue des Carrières**
**F-57050 Plappeville (FR)**

(72) Inventeur: **Etienne, Abel**
**29, rue des Carrières**
**F-57050 Plappeville (FR)**

(74) Mandataire: **Aubertin, François**
**Cabinet Lepage & Aubertin Innovations et**
**Prestations 4, rue de Haguenau**
**F-67000 Strasbourg (FR)**

Courier Press, Leamington Spa, England.

## Description

L'invention concerne une chaussette de contention, notamment un tuteur contentif de l'axe anatomo-pathologique formé par la voûte plantaire, le tendon d'Achille, les muscles ischio-jambiers, les adducteurs, le pubis, la charnière dorso-lombaire, le bassin et adapté aux fonctions concomitantes de maintien, de soutien, d'épargne, de compensation, de protection, de renfort au service des formations musculaires, articulaires, vasculaires et tendineuses.

On connaît déjà, par le document FR-A-2.347.922, un bas thérapeutique perfectionné ayant un pied circonférentiellement élastique et une jambe circonférentiellement élastique qui s'étend vers le haut à partir de la cheville. Le pied et la jambe sont réalisés en un tissu tricoté contenant des fils élastomères et la jambe du bas exerce une force de compression sur la jambe de l'utilisateur qui diminue progressivement de la cheville vers la partie supérieure du bas, tout en écartant une force de compression réduite dans la région du genou. Ce bas thérapeutique est uniquement destiné à diminuer le risque de thrombose et d'embolie et ainsi ne concerne que la circulation du sang dans les jambes. Cependant, le but de ce brevet est de pourvoir le bas thérapeutique d'un pied et d'une bande en matière antidérapante fixée sous le pied de l'utilisateur en vue de réduire tout risque de glissement de ce dernier sur le plancher.

Par ailleurs, on connaît déjà, par le document FR-A-1 111 157, l'utilisation de bourrelets à placer entre la jambe et une enveloppe de jambe. Cette dernière est une plaque en matière mousseuse avec les bords longitudinaux amincis qui sont placés de façon que leurs lèvres se chevauchent autour de la jambe. Deux bourrelets sont fixés à l'enveloppe qui sont placés au côté extérieur ou intérieur de la jambe. L'un de ces bourrelets, destiné à être placé au côté extérieur de la jambe, est en forme de "J" et consiste en une partie longue et un crochet qui soutient la cheville par l'arrière et par en-dessous. L'autre bourrelet, destiné à être placé au côté inférieur de la jambe, a une forme allongée et montre dans toute sa longueur un boudin convexe en section, sur le côté dirigé vers la jambe qui, à l'extrémité près du pied, a une courbe en forme de crochet destiné ici à être placé derrière et sous la cheville.

Toutefois, lesdits bourrelets sont destinés uniquement à soumettre à une pression accrue certaines parties de la jambe où des états de gonflement chronique occasionnent les phénomènes pathologiques les plus caractéristiques, tels que les troubles de refoulement des sucs de l'organisme, et pour empêcher des rechutes.

Or, ces moyens disparates ne peuvent convenir au but recherché par la présente invention. En effet, une athlopathie épidémique règne sur les amarres et les charnières des membres inférieurs des humains et plus particulièrement des sportifs. Dans certaines disciplines sportives, notamment le football, elle y sévit à l'état endémique pratiquement en permanence. Il s'agit d'un mal inflammatoire schléro-fibreux et d'un surmenage chronique dont le substratum est la fibre ou la cellule des systèmes nobles de l'appareil locomoteur, donc de l'appareil permettant de se mouvoir, à savoir l'appareil musculaire, ligamentaire et tendineux auxquels s'associe en permanence l'appareil vasculaire.

Cette athlopathie épidémique est due à certaines causes. Une des premières causes se rapporte à l'ambiance de vie, à savoir les conditions d'entraînement, "l'atmosphère" hygiénodiététique tout au long de l'année et spécifiquement dans la période immédiate post-compétition. Ainsi, le sportif a une mauvaise hygiène, une mauvaise diététique qui, dans les périodes suivant immédiatement l'effort intense, ne le mettent pas dans les conditions idéales de récupération. Une autre cause réside dans les aires d'évolutions tels que les sols durs, les sols synthétiques et autres. Les équipements inadaptés peuvent également être une cause de ce mal. Ces équipements existant actuellement sur le marché sont des chevillères, des bandages élastiques et adhésifs de maintien strangulant, des éléments soi-disant protecteurs mais qui, en réalité, sont agressifs et traumatisants à long terme, des chaussures sans talon ou très peu de talon qui ne tiennent compte ni de la dynamique, ni de la statique plantaire mais seulement de la mode. Il en est de même pour "les terrains minés". Il s'agit de micro-traumatismes répétés dont l'éradication ne peut être envisagée, de l'efflorescence cachée de foyers inflammatoires mal étiquetés. Un autre terrain miné consiste en des déviations métaboliques (diététiquement: l'acide urique en est un exemple frappant). Ceci est imputable du fait que les sportifs se nourrissent mal et finissent d'engendrer au sein de leur organisme des dépôts d'éléments toxiques qui ne sont pas éliminés. En effet, après un exercice intense, alors que la cellule a besoin d'une récupération avec un environnement et une ambiance idéale par l'apport d'oxygène, les sportifs ont tendance de fêter leur victoire en absorbant une boisson alcoolique, donc de faire apport de nouveaux éléments toxiques alors que cette boisson alcoolique ne devrait être prise que lorsque l'organisme est en plein repos et a parfaitement récupéré. Un autre terrain miné consiste en des malformations non diagnostiquées ou négligées, ou des mauvaises habitudes à tous les niveaux. Une autre cause est provoquée par le travail de sape de la mise en décharge d'un groupe musculaire, d'un tendon, à fortiori d'une articulation, en réponse à un traumatisme bénin ou récent mais contrôlé par le subconscient (contracture locorégionale ou à distance, contro-latérale ou sus et sous-jacente). Ainsi, lorsqu'il y a un traumatisme au niveau d'un pied, d'une cuisseou d'un élément quelconque d'un membre, il se forme une compensation exercée par le membre contro-latéral. Cette compensation, se faisant inconsciemment pour épargner en partie le labeur du membre traumatisé, provoque un surcroît de travail du membre contro-latéral, ce qui entraîne pour ce dernier la création de phénomènes de contraction. De ce fait, pour la récupération au niveau du membre malade, il ne faut pas seulement tenir compte de ce membre malade mais également du membre qui prend en charge ledit membre malade.

En conclusion, tous ces facteurs induisent, favorisent, entretiennent, fixent, sur une orbite privilégiée de chronicité, le délabrement organique du sportif à moyen et à long terme. Les muscles, vaisseaux, articulations, tendons, ligaments, qui sont autant d'éléments de nature différente, sont solidaires et indépendants physiologiquement et pathologiquement, Tous ces éléments ne travaillent pas pour leur propre compte mais sont en relation les uns avec les autres soit dans les conditions normales, soit dans les conditions de traumatismes ou de blessures. Ainsi, lorsqu'un muscle est malade, forcément il y a un tendon qui souffre; quand un tendon souffre, forcément il y a une art'iculation qui participe etc.... Ceci entraîne une souffrance au niveau de la circulation qui doit apporter une oxygénation supplémentaire due au déficit du muscle, du tendon, de l'articulation....

C'est ainsi que, sans ordre ni préférence particulière, la voûte plantaire, le tendon d'Achille, les muscles ischio-jambiers, donc les muscles allant de la jambe vers la cuisse, les adducteurs, le pubis, la charnière dorso-lombaire et le bassin forment une entité c'est-à-dire un tout dont les composants sont ipso-facto indissociables surtout au plan thérapeutique. C'est pourquoi, lorsqu'un organe est malade et/ou présente une atteinte quelconque et que l'on envisage de la traiter par une action liée, on fait une erreur car il est nécessaire de prendre en compte tout l'ensemble. Cependant, la preuve de la non-application et de l'absence de cette considération élémentaire de l'interdépendance fondamentale est démontrée quotidiennement.

La présente invention a pour but de remédier à ces inconvénients. L'invention telle qu'elle est caractérisée dans la revendication, résout le problème consistant à créer une chaussette de contention, notamment un tuteur contentif de l'axe anatomo-pathologique formé par la voûte plantaire, le tendon d'Achille, les muscles ischiojambiers, les adducteurs, le pubis, la charnière dorso-lombaire, le bassin et adapté aux fonctions concomitantes de maintien, de soutien, d'épargne, de compensation, de protection, de renfort au service des formations musculaires, articulaires, vasculaires et tendineuses qui, s'étendant depuis une zone de recouvrement total des orteils jusqu'à la jambe dans leur intégralité, comporte en combinaison un tissu composé de fibres élastiques souples non constrictives et très dégressives et doublé d'un tissu d'une matière lisse absorbante et lavable, et présentant des oeillets d'aération et de traction sur les parties latérales hautes et basses du segment jambier et une bande élastique sous-gonale, ce tissu pourvu d'une circonférence supérieure s'étendant depuis l'avant juxtaratolien jusqu'à l'arrière de la partie basse du triangle supérieur du creux poplite, comprenant, noyés dans son épaisseur, deux coussinets en forme de haricot comblant les gouttières rétro-malléolaires situées de part et d'autre du corps du tendon d'Achille pour l'étançonnement de ce dernier, une talonnette biseautée d'une hauteur de quelques millimètres, et de préférence de l'ordre de cinq millimètres, en matière plastique telle que le polyéthylène ébauchant un discret échinisme et antéversant l'axe jambier pour l'allègement de la tension achilléenne et un renfort en forme de chevillère et présentant, au niveau du complexe ostéo-musculo-tendineux, une zone d'élasticité de transition s'étendant de la partie moyenne du pied en passant par la jonction du tiers inférieur jusqu'aux deux tiers supérieurs de la jambe.

Les avantages obtenus grâce à cette invention consistent essentiellement en ce que ladite chaussette apparait comme une chaîne contentive composée d'éléments indissociables. En effet, elle intéresse le pied et la jambe dans leur intégralité. La circonférence supérieure est juxtaratolienne en avant, elle empiète en arrière sur la partie basse du triangle supérieur du creux poplité, évitant ainsi toute compression vasculaire à la "pliure" et le glissement, notamment lorsqu'elle s'arrête en regard de la partie haute des mollets hypertrophiques.

Par ailleurs, la chaussette de contention est spécifique dans son action de favoriser la circulation veineuse. Simultanément, à cette action, la chaussette augmente la dynamique de la voûte plantaire, en même temps qu'elle maintient électivement le tendon d'Achille, en même temps qu'elle respecte le flux sanguin de retour, en même temps qu'elle s'adresse, globalement considérée, à une pathologie spécifique.

Cette chaussette est édifiée dans le but de parvenir à s'opposer par l'ensemble de ses éléments bien particuliers, fondamentaux et indissociables qui le composent à l'installation et au maintien de troubles bien spécifiques et qui comprennent essentiellement le "shin-splint".

L'invention est exposée ci-après plus en détail à l'aide de dessins représentant seulement un mode d'exécution.

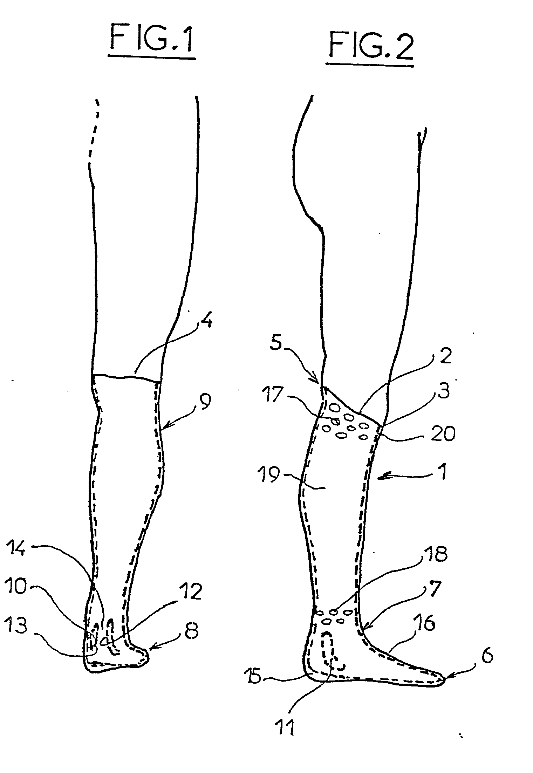

La figure 1 représente une vue postérieure d'un membre inférieur pourvu de la chaussette de contention conforme à l'invention.

La figure 2 représente une vue latérale externe de ce membre inférieur.

La chaussette 1 est un matériel prophylactique donc de prévention et thérapeutique d'un certain nombre d'affections et d'atteintes. Une première affection et/ou atteinte se rapporte à un ensemble de manifestations subjectives ressenties du couple jambe-pied et ceci sans atteinte organique focalisée précise et où les souffrances vasculaires, musculaires, tendineuses, ostéopériostées, articulaires sont intimement liées et associées dans une pathologie de toute évidence inflammatoire et d'insuffisance circulatoire. Ainsi, on obtient un état de souffrance globale déclenché par un ensemble sans pouvoir préciser l'élément en cause. Une seconde affection et/ou atteinte consiste en une inflammation soit post-contusionnelle, soit de surmenage provoquée par des entraînements intempestifs dans de mauvaises conditions dues à des revêtements synthétiques, à des sols durs saisonniers: hiver comme été, soit inflammation plus précisément musculo-tendineuse par prise en charge excessive de compensation d'un muscle ou d'un segment de membre controlatéral. Une autre affection et/ou atteinte consiste en l'insuffisance ou souffrance circulatoire se situant indifféremment au niveau des sphères veineuses artérielles ou capillaires de voisinage.

A cet effet, la circonférence supérieure 2 de la chaussette 1 est à l'avant 3 juxtaratolien alors qu'à l'arrière 4 elle empiète en arrière sur la partie basse 5 du triangle supérieur du creux poplité. De ce fait, on évite ainsi toute compression vasculaire à la "pliure" et le glissement de la chaussette 1, notamment lorsqu'elle s'arrête en regard de la partie haute des mollets hypertrophiques.

La chaussette 1 est composée de fibres élastiques souples non constrictives et très dégressives depuis l'extrémité des orteils. Ladite chaussette 1 comporte une zone de recouvrement total 6 des orteils. De ce fait, on supprime toute hypercompression à la base des orteils par rétraction des contentions qui sont ouvertes classiquement aux extrémités. En effet, toutes les contentions élastiques de jambe sont en règle générale ouvertes du côté des orteils. Ainsi, au niveau de la racine des orteils, on exerce une rétraction de la partie antérieure du pied. Cette rétraction devient constrictive au niveau de la racine des orteils et provoque une gêne circulatoire importante donc phénomène d'ankylose. Celle-ci indispose le porteur de la contention et l'oblige bien souvent à l'enlèvement de la chaussure et la remise en place de la partie antérieure de la chaussette pour supprimer la striction en question.

La chaussette 1 comporte un renfort 7 en forme de chevillère au niveau du complexe ostéo-musculo-tendineux de la cheville mais respectant une élasticité de transition pour éviter toute incidence fâcheuse sur les circulations d'apport et de retour, source inéluctable d'inconfort, de fourmillements, de lourdeurs, de crampes, autant de manifestations subjectives génératrices de contre-performances inexpliquées. En effet, si la contention ne comportait pas cette élasticité de transition, il y a risque de souffrance circulatoire en raison de zones de striction opérant au niveau de certains endroits de la jambe engendrant donc une indisposition globale.

Cette zone d'élasticité de transition s'étend de la partie moyenne 8 du pied en passant par la jonction du tiers inférieur jusqu'aux deux tiers supérieurs 9 de la jambe.

Par ailleurs, il existe également des altérations au niveau du tendon d'Achille, notamment les tendinites du corps, constitué par la partie principale du tendon et comprenant tout son trajet anatomique et son attache, les bursites difficilement décelables et qui correspondent à l'inflammation d'une bourse séreuse située entre le tendon d'Achille et la partie haute de la face postérieure du calcanéum, la simple lésion par contusion, les fissures ou ruptures partielles, récentes ou anciennes ainsi que les séquelles de rupture totale traitées chirurgicalement initialement.

A cet effet, la chaussette 1 comprend, noyés dans son épaisseur, des moyens de soutien. Ces moyens de soutien sont deux coussinets 10, 11 en forme de haricot comblant les gouttières rétromalléolaires 12, 13 de part et d'autre du corps 14 du tendon d'Achille. Ces coussinets 10, 11 assurent l'étançonnement et l'épargne de mouvements de vibrations ou de latéralité occasionnés soit par la dureté des sols, soit par la dynamique musculaire excessive due à un surentraînement, un surmenage ou autres. Ces coussinets 10, 11 épousent rigoureusement l'anatomie des deux dépressions, bloquant ainsi tout frottement de la chaussette 1 de contention sur la face postérieure du tendon. Un autre moyen de soutien consiste en une talonnette biseautée 15 en polyéthylène de quelques millimètres de hauteur, par exemple de l'ordre de cinq millimètres ayant pour but d'alléger la tension achilléenne en ébauchant un discret échinisme et en antéversant l'axe jambier. Ainsi, on procède à une décontraction du tendon pour éviter une hyperextension pouvant provoquer des douleurs au niveau des muscles du mollet et/ou de la cuisse.

En outre, la chaussette 1 sert de matériel prophylactique et thérapeutique envers:

les contractures, contusions, claquages, élongations, déchirures musculaires.

les myosites simples ou ossifiantes.

les hématomes bénins ou graves, circonscrits ou diffus, à plus forte raison calcifiés ou enkystés.

les épiphysites et périostites notamment prétibiales.

les séquelles d'entorses, bénignes ou graves, plâtrées ou non.

les raideurs, les ankyloses post-traumatiques et d'immobilisation.

les insuffisances circulatoires de support lympho-capillaro-veineux.

C'est pourquoi, la chaussette 1 comporte également un relâchement et une diminution de l'épaisseur des fibres constrictives au niveau 16 du cou-de-pied, excluant ainsi toute irritation des tendons antérieurs.

Le tissu de ladite chaussette 1 est doublé d'une matière lisse, absorbante et lavable permettant une mise en place facile et une atténuation de l'hyperhidrose soit constitutionnelle, soit provoquée par le matériel de contention en général.

Par ailleurs, la chaussette 1 comporte des oeillets d'aération et de traction 17, 18 sur les parties latérales hautes et basses du segment jambier 19.

Pour éviter une souffrance de la circulation de retour, il est souhaitable d'absorber sans striction, les variations des profils anatomiques du mollet. A cet effet, la chaussette 1 comporte une bande élastique sousgonale 20.

Dans toutes les indications citées ci-dessus, la chaussette selon l'invention est un complément thérapeutique indispensable, notamment pour toutes les lésions et/ou anomalies apparentes ou démontrées qui touchent l'axe locomoteur.

**Revendication**

Chaussette de contention, notamment tuteur contentif de l'axe anatomo-pathologique formé par la voûte plantaire, le tendon d'Achille, les muscles ischio-jambiers, les adducteurs, le pubis, la charnière dorso-lombaire, le bassin et adapté aux fonctions concomitantes de maintien, de soutien, d'épargne, de compensation, de protection, de renfort au service des formations musculaires, articulaires, vasculaires et tendineuses qui, s'étendant depuis une zone de recouvrement total des orteils (16) jusqu'à la jambe dans leur intégralité, elle comporte, en combinaison, un tissu composé de fibres élastiques souples non constrictives et très dégressives et doublé d'un tissu d'une matière lisse absorbante et lavable, et présentant des oeillets d'aération et de traction (17, 18) sur les parties latérales hautes et basses du segment jambier (19) et une bande élastique sous-gonale (20), ce tissu pourvu d'une circonférence supérieure (2) s'étendant depuis l'avant juxtaratolien (3) jusqu'à l'arrière (4) de la partie basse (5) du triangle supérieur du creux poplité, comprenant, noyés dans son épaisseur, deux coussinets (11, 12) en forme de haricot comblant les gouttières rétro-malléolaires situées de part et d'autre du corps du tendon d'Achille pour l'étançonnement de ce dernier, une talonnette biseautée (15) d'une hauteur de quelques millimètres, et de préférence de l'ordre de cinq millimètres, en matière plastique telle que le polyéthylène ébauchant un discret échinisme et antéversant l'axe jambier pour l'allègement de la tension achilléenne et un renfort (7) en forme de chevillère et présentant, au niveau du complexe ostéo-musculo-tendineux, une zone d'élasticité de transition s'étendant de la partie moyenne (8) du pied en passant par la jonction du tiers inférieur jusqu'aux deux tiers supérieurs (9) de la jambe.

**Patentanspruch**

Stützstrumpf, die nämlich die von dem Fußsohlengewölbe, der Achillessehne, den Ischiasbeinmuskeln, den Adduktoren, dem Schambein, dem Rückenkreuzgelenk, dem Becken gebildete anatomisch-pathologische Achse stützt und für die gleichzeitigen Haltungs-, Unterstützungs-, Ersparnis-, Ausgleich-, Schutz- und Verstärkungsfunktionen der Muskel-, Gelenk-, Gefäß- und Sehnenstrukturen geeignet ist, der sich von einem Bereich (16), der die Zehen bis zum Bein völlig abdeckt, erstreckt und, in Kombination, ein Gewebe aus nichtverengenden und sehr abnehmenden, weichen, elastischen Fasern und mit einem Gewebe aus saugendem und waschbarem, glattem Material gefuttert ist, umfaßt, das Lüftungs- und Ziehöffnungen (17, 18) an den oberen und unteren seitlichen Teilen des Beinteils (19) und ein elastisches Band (20) unter dem Knie aufzeigt, wobei sich dieses mit einem oberen Umkreis (2) versehene Gewebe von der Juxtaratolischen Vorderseite (3) bis zur Hinterteil (4) des Unterteils (5) des oberen Dreiecks der Kniekehlenhöhle ausstreckt, zwei bohnenförmige, in dessen Dicke versenkte Kissen (ll, 12), welche die sich an beiden Seiten des Achillessehnenkörpers befindlichen Retromalleusrinnen füllen, zur Unterstützung dieser Sehne, eine schräge Ferse (15) einer Höhe von ein Paar Millimeter, vorzugsweise im Bereich von fünf Millimeter, aus Kunststoff, wie Polyäthylen, die eine diskrete Rückgratgestaltung aufweist und die Beinachse nach vorne neigen läßt, um die Achillesanstrengung zu mildern, und eine knöchelstückförmige Verstärkung (7) umfaßt und im Bereich des Osteomuskularen Sehnenkomplexes eine Übergangselastizitätszone aufweist, die sich vom Mittelteil (8) des Fußes, über das Gelenk des unteren Drittels, bis zu den oberen zwei Dritteln (9) des Beines ausstreckt.

**Claim**

Support stocking for the lower limb, viz, supporting the anatomicpathological axis formed by the plantar arch, the Achilles tendon, the ischiotibial muscles, the adductors, the pubis, the dorsolumbar hinge-joint, the pelvis and adapted to the concomitant functions of maintaining, supporting, saving, compensating, protecting, reinforcing for the muscular, articular, vascular and tendinous structures which, extending from an area of total recovering of the toes (16) up to the leg in their whole, comprises, in combination, a fabric of non-constrictive and very degressive flexible elastical fibres and lined with a fabric of absorbing and washable smooth material and having aeration and traction holes (17, 18) at the upper and lower side parts of the leg segment (19) and a strip under the knee (20), this fabric provided with an upper circumference (2) extending from the front juxtaratolian (3) to behind the lower part (5) of the upper triangle of the popliteal recess, comprising, sunk into its thickness, two bean-shaped cushings (11, 12) filling the retromalleolar gutters located on both sides of the Achilles tendon for propping same, a bevelled heel-piece (15) with a height of some millimeter, and preferably of about five millimeter, of a plastic material such as polyethylene and forming a discrete echinus and anteversing the leg axis to relieve the achillean stress and a peg-shaped reinforcement (7) having, in the area of the osteo-musculartendinous complex, a transition elasticity area extending from the central part (8) of the foot, through the joint of the lower third, to the upper two-thirds (9) of the leg.

# FIG.1

# FIG.2